# EUROPEAN PATENT SPECIFICATION

(11) **EP 2 786 854 B1**
(45) Date of publication and mention of the grant of the patent: **14.02.2018**
(21) Application number: 12853841.0
(22) Date of filing: 20.02.2012
(51) Int. Cl.: A61L 27/18, B29C 43/02

(54) **PROCESS UTILIZING PEEK MATERIAL TO MANUFACTURE CEREBRAL CRANIUM BONE AND SPECIAL EQUIPMENT**
VERFAHREN UNTER VERWENDUNG EINES PEEK-MATERIALS ZUR HERSTELLUNG EINES ZEREBRALEN SCHÄDELKNOCHENS UND SPEZIALAUSRÜSTUNG DAFÜR
PROCÉDÉ UTILISANT DE LA MATIÈRE PEEK POUR FABRIQUER UN OS CRÂNIO-CÉRÉBRAL ET ÉQUIPEMENT SPÉCIAL

(30) Priority: 29.11.2011 CN 201110386538
(43) Date of publication of application: 08.10.2014
(73) Proprietor: Wuhan Constant Science and Technology Ltd., Hubei 430022 (CN)
(72) Inventor: HUANG, Dengfeng, Wuhan Hubei 430022 (CN)
(74) Representative: Laufhütte, Dieter
(86) International application number: PCT/CN2012/071370
(87) International publication number: WO 2013/078790

(56) References cited:
- EP-A1- 0 479 257
- EP-A1- 1 813 292
- WO-A1-2011/036148
- WO-A2-2011/072212
- CN-A- 1 593 670
- CN-A- 101 092 059
- CN-A- 101 883 535
- CN-U- 2 061 891

## Description

### FIELD OF THE INVENTION

The invention relates to a method of preparing bones of cerebral cranium using PEEK (polyether ether ketone), and also relates to a device for use in the above method, as defined in the appended claims.

### BACKGROUND OF THE INVENTION

Current artificial skull defects are mainly repaired by a titanium mesh plate which has high strength, non-toxicity, and excellent biocompatibility with human bodies. However, as a metal material, electrochemical reaction of the titanium mesh plate easily occurs in the human body, thereby resulting in electrochemical corrosion. When the titanium mesh stays in the human body for a long period, the surrounding human tissue turns black or even results in necrosis. Meanwhile, the surface of the titanium mesh plate becomes uneven due to the electrochemical corrosion, which is not beneficial for the human body after a long period of the implantation.

Chinese patent application No. 200410012852.9 discloses a composite artificial skull includes polymethyl methacrylate (PMMA), a biological carbon fiber or glass fiber, and hydroxyapatite powder or β- tricalcium phosphate powder, and is prepared by multi-layers cladding and heat pressing technology. However, the biological carbon fiber or glass fiber has not yet been certificated as a human implantation material and is still in its research period.

Good human implant material has always been developing as an artificial brain skull material.

Polyether ether ketone (PEEK) resin is a special engineering plastic that has an excellent performance and superior to other special engineering plastics in many aspects. PEEK and the compound thereof is high temperature resistant and have a glass transition temperature of 143°C and a fusion point of 343°C. Independent testing results show that PEEK has a highest heat distortion temperature of 315°C (filled with glass fiber) and a long-term use temperature of 260°C. PEEK has excellent mechanical performance, good self-lubrication, chemical corrosion resistance, stable insulation performance, flame retardancy, peel resistance, irradiation resistance, X rays permeability, hydrolysis resistance, and easy processing.

Mechanical performance of PEEK resin is similar to aluminum alloy. It is capable of resisting almost all solvents except concentrated sulfuric acid, maintaining its characteristics in the condition of a high dose of γ -ray irradiation, and still maintaining its superb physical and chemical properties and mechanical performance at the temperature of 180°C. Besides, PEEK resin is advantageous in light weight, non-toxicity, and corrosion resistance.

In China and the United States Food and Drug Administration, PEEK has been recognized as human implant material (e. g. for intervertebral implant materials, etc.). A typical method for processing PEEK to form a human implantation material is the mechanical cold method which costs a large amount of PEEK material, approximately 70 and 90 wt. % or even more. Besides, the PEEK implantation material only produced in Britain, whichis highly priced, about 300,000 RMB/kg.

Therefore, the research of PEEK material as artificial skull and the research of PEEK material processing methods as artificial skull have become an urgent and important work. EP 1 813 292 A1 relates to a surgical implant having a body comprising polyaryletherketone and a method for manufacturing a surgical implant having a body comprising polyaryletherketone.

### SUMMARY OF THE INVENTION

In view of the above-described problems, it is one objective of the invention to provide a method for preparing bones of cerebral cranium, in which, thermal forming and cold processing are combined and thus raw material is saved.

It is another objective of the invention to provide a device for preparing bones of cerebral cranium.

To achieve the above objective, in accordance with one embodiment of the invention, there is provided a method for preparing bones of cerebral cranium. The method, which is also defined in claim 1, comprises the following steps:
a) selecting polyetheretherketone (PEEK) as a raw material according to computed tomography (CT) data of a patient;
b) heating the PEEK material in a heating device to reach a softening point of 260±10°C;
c) hot pressing the heated PEEK material obtained in step b) in a forming die, cooling and shaping the PEEK material to yield a blank;
d) curing the blank, and placing the blank in a thermostat for removal of internal stress and resilience;
e) removing surface crystals and impurities of the blank resulting from the hot pressing;
f) mechanically processing the blank according to the CT data of the patient to yield a product having desired size and shape; and
g) washing, disinfecting, and packaging the product.

The heating device discriminatively heats different areas of the PEEK material to meet the requirements of temperature and strain force for material deformation according to the CT data of the patient, areas requiring a relatively large deformation are heated at a relatively high temperature, and areas requiring a relatively small deformation is heated at a relatively low temperature.

The forming die comprises an upper mold comprising an upper needle module and a lower mold comprising a lower needle module. The lower needle module is controlled by a programmable logic controller (PLC) for space regulating and locating according to the CT data. The upper needle module is also regulated correspondingly. The heated PEEK material is placed on the upper mold and processed by the upper and lower needle modules to yield the blank.

In accordance with another embodiment of the invention, and as defined in claim 2, there is provided a device for preparing bones of cerebral cranium, comprising: a forming die and a heating device. The forming die comprises an upper mold base, an upper mold, a locating device, a lower mold, a lower mold base, a base frame. The upper mold is fixed on the upper mold base. The lower mold base is fixed on the base frame. The lower mold is fixed on the lower mold base, and a lower end of the locating device is fixed on the base frame. The upper mold base is fixed on the locating device, and a rear end of the upper mold base is located in a sliding groove of the locating device. The upper mold and the lower mold each comprises a plurality of needle modules. The needle modules are controlled by a first PLC via a displacement sensor.

The locating device is perpendicular to the base frame.

The lower mold base is fixed on the base frame via a pin bolt.

The heating device comprises an upper cover plate, an upper heater, a support bracket, a lower heater, and a base. The support bracket is disposed between the upper heater and the lower heater. The upper heater is disposed under a central area of the upper cover plate. The lower heater is disposed over a central area of the base. The upper heater and the lower heater each comprises a plurality of independent little resistance wires, and a second PLC is connected to the little resistance wires via a temperature sensor.

The invention further provides a method for preparing bones of cerebral cranium comprising employing PEEK as a raw material, as defined in claim 1.

Advantages according to embodiments of the invention are summarized as follows. The method combines the thermal forming and cold processing thereby significantly reducing the PEEK material wastage and saving the medical expenses. The resulting products are made according to the CT data of patients and thus can be tightly integrated with human skull, showing good biocompatibility, and no rejection and side effect are observed.

### BRIEF DESCRIPTION OF THE DRAWINGS

FIG. 1 is a schematic diagram of a device for preparing bones of cerebral cranium according to one embodiment of the invention;
FIG. 2 is a top view of a device for preparing bones of cerebral cranium in the absence of an upper mold according to one embodiment of the invention;
FIG 3 is a block diagram showing a procedure control of shaping PEEK material for preparing bones of cerebral cranium; and
FIG. 4 is a block diagram showing a procedure control of heating PEEK material for preparing bones of cerebral cranium.

### DETAILED DESCRIPTION OF THE EMBODIMENTS

For further illustrating the invention, experiments detailing a method and device preparing bones of cerebral cranium are described below. It should be noted that the following examples are intended to describe and not to limit the invention.

The invention provides a method for preparing bones of cerebral cranium. The method comprises:
a) selecting polyetheretherketone (PEEK) as a raw material according to computed tomography (CT) data of a patient;
b) heating the PEEK material in a heating device to reach a softening point of 260±10°C;
c) hot pressing the heated PEEK material obtained in step b) in a forming die, cooling and shaping the PEEK material to yield a blank;
d) curing the blank, and placing the blank in a thermostat for removal of internal stress and resilience;
e) removing surface crystals and impurities of the blank resulting from the hot pressing;
f) mechanically processing the blank according to the CT data of the patient to yield a product having desired size and shape; and
g) washing, disinfecting, and packaging the product.

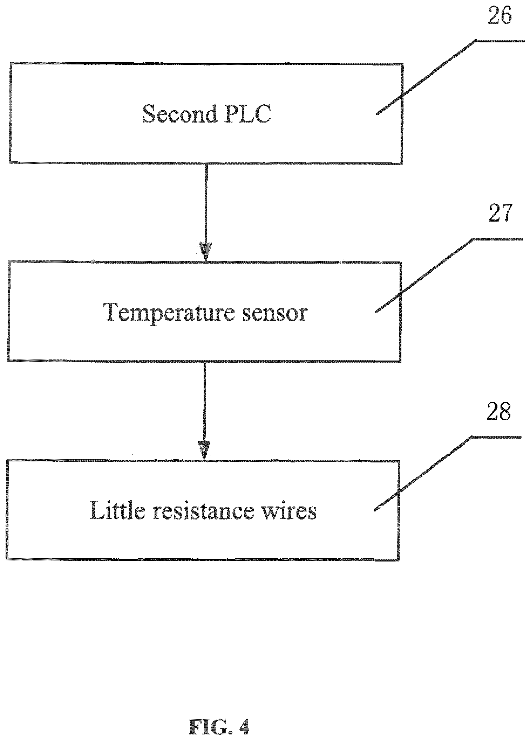

The heating device discriminatively heats different areas of the PEEK material to meet the requirements of temperature and strain force for material deformation according to the CT data of the patient, areas requiring a relatively large deformation are heated at a relatively high temperature, and areas requiring a relatively small deformation is heated at a relatively low temperature (the heating is controlled through a second PLC, a temperature sensor, and little resistance wires, as shown in FIG. 4).

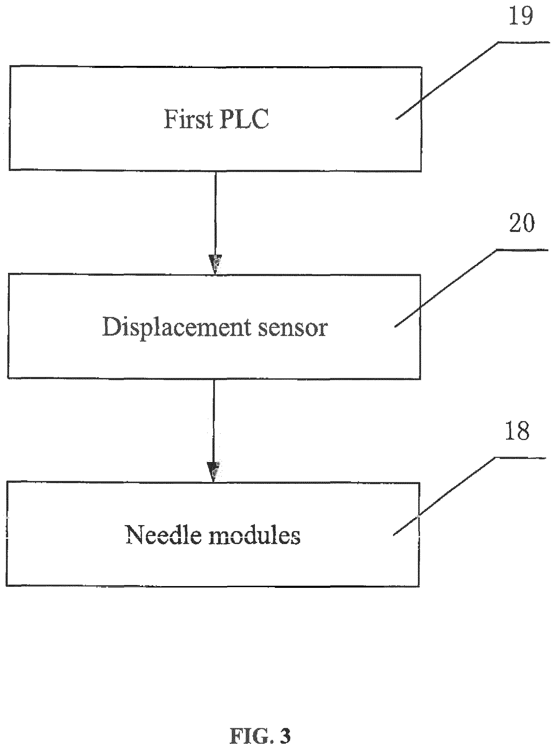

The forming die comprises an upper mold comprising an upper needle module and a lower mold comprising a lower needle module. The lower needle module is controlled by a programmable logic controller (PLC) for space regulating and locating according to the CT data. The upper needle module is also regulated correspondingly, and thus the heated PEEK material is placed on the upper mold and processed by the upper and lower needle modules to yield the blank (as shown in FIG. 3).

A device for preparing bones of cerebral cranium comprises: a forming die and a heating device. The forming die comprises an upper mold base 11, an upper mold 12, a locating device 13, a lower mold 14, a lower mold base 15, and a base frame 16. The upper mold 12 is fixed on the upper mold base 11. The lower mold base 15 is fixed on the base frame 16. The lower mold 14 is fixed on the lower mold base 15, and a lower end of the locating device 13 is fixed on the base frame 16. The upper mold base 11 is fixed on the locating device 13, and a rear end of the upper mold base 11 is located in a sliding groove of the locating device 13. The upper mold 12 and the lower mold 14 each comprises a plurality of needle modules 18. The needle modules 18 are controlled by a first PLC 19 via a displacement sensor 20.

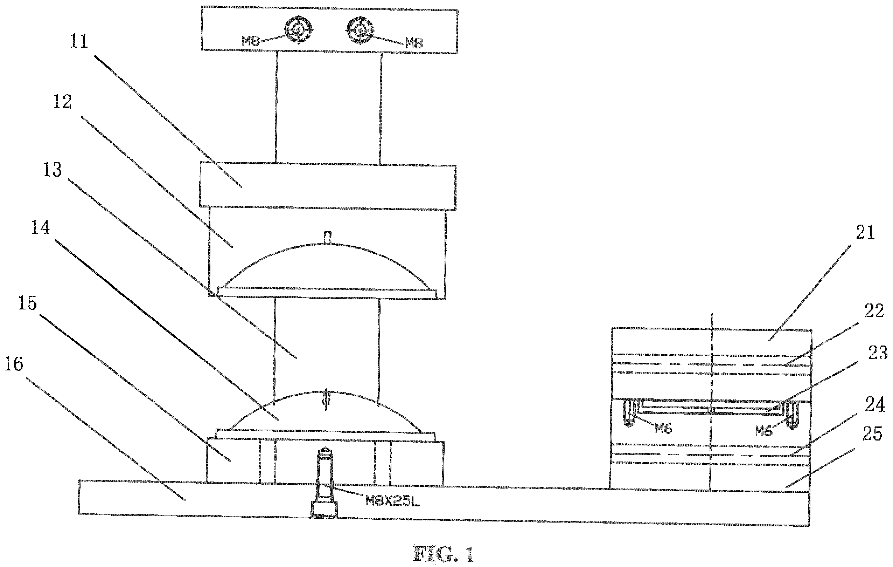

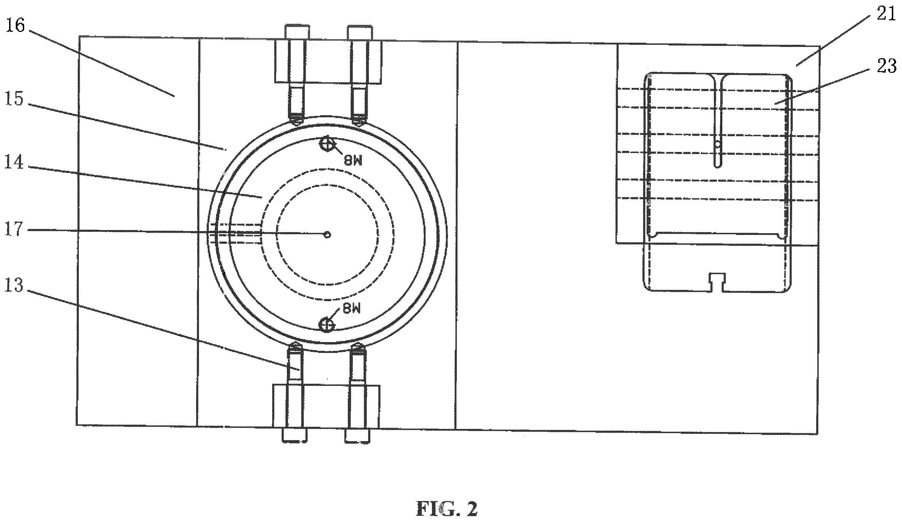

The locating device 13 is perpendicular to the base frame 16. The lower mold base 15 is fixed on the base frame 16 via a pin bolt 17. The heating device comprises an upper cover plate 21, an upper heater 22, a support bracket 23, a lower heater 24, and a base 25. The support bracket 23 is disposed between the upper heater 22 and the lower heater 24. The upper heater 22 is disposed under a central area of the upper cover plate 21, and the lower heater 24 is disposed over a central area of the base 25. The upper heater 22 and the lower heater 24 each comprises a plurality of independent little resistance wires 28, and a second PLC 26 is connected to the little resistance wires 28 via a temperature sensor 27 (as shown in FIGS. 1-4).

### Example

Five healthy adult dogs without skull defects were sampled. Take CT photos from different sample points of the skulls of the five dogs, and collect the CT data. Thereafter, the following steps were carried out: a) selecting polyetheretherketone (PEEK) as a raw material according to computed tomography (CT) data of a patient;
b) heating the PEEK material in a heating device to reach a softening point of 260±10°C;
c) hot pressing the heated PEEK material obtained in step b) in a forming die, cooling and shaping the PEEK material to yield a blank;
d) curing the blank, and placing the blank in a thermostat for removal of internal stress and resilience;
e) removing surface crystals and impurities of the blank resulting from the hot pressing;
f) mechanically processing the blank according to the CT data of the patient to yield a product having desired size and shape; and g) washing, disinfecting, and packaging the product.

The heating device discriminatively heats different areas of the PEEK material to meet the requirements of temperature and strain force for material deformation according to the CT data of the patient, areas requiring a relatively large deformation are heated at a relatively high temperature, and areas requiring a relatively small deformation is heated at a relatively low temperature. The heating is controlled through a second PLC, a temperature sensor, and little resistance wires, as shown in FIG. 4.

The forming die comprises an upper mold comprising an upper needle module and a lower mold comprising a lower needle module. The lower needle module is controlled by a programmable logic controller (PLC) for space regulating and locating according to the CT data. The upper needle module is also regulated correspondingly. The heated PEEK material is placed on the upper mold and processed by the upper and lower needle modules to yield the blank (as shown in FIG. 3).

The five dogs were performed with craniotomy, respectively, at the preset sample points of the skulls. The parts of the skulls were taken out and replaced by the PEEK bones of cerebral cranium. Thereafter, the soft tissue was refilled and epidermis sutured, both were confirmed by CT examination. Observe the recovery of the dogs in the first month, the third month, and the sixth month. The results showed that no heterotopia, deformation, and rejection reaction occurred. Six months later, the five dogs recovered as well as normal dogs.

## Claims

1. A method for preparing bones of cerebral cranium, the method comprising:
a) selecting polyetheretherketone (PEEK) as a raw material according to computed tomography (CT) data of a patient;
b) heating the PEEK material in a heating device to reach a softening point of 260±10°C;
c) hot pressing the heated PEEK material obtained in step b) in a forming die, cooling and shaping the PEEK material to yield a blank;
d) curing the blank, and placing the blank in a thermostat for removal of internal stress and resilience;
e) removing surface crystals and impurities of the blank resulting from the hot pressing;
f) mechanically processing the blank according to the CT data of the patient to yield a product having desired size and shape; and
g) washing, disinfecting, and packaging the product;
the heating device discriminatively heats different areas of the PEEK material to meet the requirements of temperature and strain force for material deformation according to the CT data of the patient, areas requiring a relatively large deformation are heated at a relatively high temperature, and areas requiring a relatively small deformation is heated at a relatively low temperature;
the forming die comprises an upper mold comprising an upper needle module and a lower mold comprising a lower needle module, the lower needle module is controlled by a programmable logic controller (PLC) for space regulating and locating according to the CT data, the upper needle module is also regulated correspondingly, the heated PEEK material is placed on the upper mold and processed by the upper and lower needle modules to yield the blank.

2. A device for preparing bones of cerebral cranium, comprising: a forming die and a heating device, **characterized in that**
the forming die comprises an upper mold base (11), an upper mold(12), a locating device (13), a lower mold(14), a lower mold base(15), a base frame(16); the upper mold(12) is fixed on the upper mold base(11); the lower mold base(15) is fixed on the base frame(16); the lower mold (14) is fixed on the lower mold base(15), and a lower end of the locating device (13) is fixed on the base frame(16); the upper mold base (11) is fixed on the locating device(13), and a rear end of the upper mold base (11) is located in a sliding groove of the locating device(13); the upper mold (12)and the lower mold (14)each comprises a plurality of needle modules(18); the needle modules (18) are controlled by a first PLC(19) via a displacement sensor(20);
the locating device (13) is perpendicular to the base frame (16);
the lower mold base(15) is fixed on the base frame(16) via a pin bolt(17);
the heating device comprises an upper cover plate(21), an upper heater(22), a support bracket(23), a lower heater(24), and a base(25); the support bracket(23) is disposed between the upper heater (22)and the lower heater(24); the upper heater (22)is disposed under a central area of the upper cover plate(21); the lower heater(24) is disposed over a central area of the base(25); the upper heater (22)and the lower heater (24)each comprises a plurality of independent little resistance wires(28), and a second PLC (26)is connected to the little resistance wires (28)via a temperature sensor(27), wherein the second PLC (26), the temperature sensor and little resistance wires control the heating device to heat the PEEK material to reach a softening point of 260±10°C.

## Patentansprüche

1. Verfahren zum Herstellen von zerebralen Schädelknochen, das Verfahren umfassend:
a) Auswählen von Polyetheretherketon (PEEK) als Ausgangsmaterial gemäß Computertomographie (CT) - Daten eines Patienten;
b) Erhitzen des PEEK-Materials in einer Heizvorrichtung, um einen Erweichungspunkt von 260 +/- 10 °C zu erreichen;
c) Heißpressen des in Schritt b) erhaltenen erhitzten PEEK-Materials in einem Umformwerkzeug, Kühlen und Formen des PEEK-Materials, so dass ein Rohling entsteht;
d) Aushärten des Rohlings, und Platzieren des Rohlings in einem Thermostat zur Beseitigung von Eigenspannung und elastischer Rückverformung;
e) Entfernen von Oberflächenkristallen und Verunreinigungen des Rohlings, die auf das Heißpressen zurückzuführen sind;
f) Mechanisches Bearbeiten des Rohlings gemäß den CT-Daten des Patienten, so dass ein Produkt entsteht, welches eine gewünschte Größe und Form besitzt; und
g) Waschen, Desinfizieren, und Verpacken des Produkts;
wobei die Heizvorrichtung unterschiedliche Bereiche des PEEK-Materials unterschiedlich erhitzt, um die Erfordernisse an Temperatur und Belastungskraft zur Materialdeformation gemäß den CT-Daten des Patienten zu erfüllen, wobei Bereiche, die eine vergleichsweise große Deformation erfordern, auf eine vergleichsweise hohe Temperatur erhitzt werden, und Bereiche, die eine vergleichsweise kleine Deformation erfordern, auf eine vergleichsweise niedrige Temperatur erhitzt werden;
wobei das Umformwerkzeug ein oberes Formwerkzeug aufweisend ein oberes Nadelmodul und ein unteres Formwerkzeug aufweisend ein unteres Nadelmodul aufweist, wobei das untere Nadelmodul von einer speicherprogrammierbaren Steuerung (PLC) zur Raumregulierung und Anordnung gemäß den CT-Daten gesteuert wird, das obere Nadelmodul ebenfalls entsprechend geregelt wird, das erhitzte PEEK-Material auf dem oberen Formwerkzeug platziert wird und vermittels der oberen und unteren Nadelmodule bearbeitet wird, so dass der Rohling entsteht.

2. Vorrichtung zur Herstellung von zerebralen Schädelknochen, aufweisend: ein Umformwerkzeug und eine Heizvorrichtung, **dadurch gekennzeichnet, dass** das Umformwerkzeug eine obere Formwerkzeugbasis (11), ein oberes Formwerkzeug (12), eine Anordnungsvorrichtung (13), ein unteres Formwerkzeug (14), eine untere Formwerkzeugbasis (15), einen Basisrahmen (16) aufweist; wobei das obere Formwerkzeug (12) an der oberen Formwerkzeugbasis (11) befestigt ist; die untere Formwerkzeugbasis (15) an dem Basisrahmen (16) befestigt ist; das untere Formwerkzeug (14) an der unteren Formwerkzeugbasis (15) befestigt ist, und ein unteres Ende der Anordnungsvorrichtung (13) an dem Basisrahmen (16) befestigt ist; die obere Formwerkzeugbasis (11) an der Anordnungsvorrichtung (13) befestigt ist, und sich ein hinteres Ende der oberen Formwerkzeugbasis (11) in einer Gleitspur der Anordnungsvorrichtung (13) befindet; das obere Formwerkzeug (12) und das untere Formwerkzeug (14) jeweils eine Vielzahl von Nadelmodulen (18) aufweisen; die Nadelmodule (18) von einer ersten speicherprogrammierbaren Steuerung (PLC) über einen Wegnehmer (20) gesteuert werden;
die Anordnungsvorrichtung (13) senkrecht zu dem Basisrahmen (16) ist; die untere Formwerkzeugbasis (15) über einen Stiftbolzen (17) an dem Basisrahmen (16) befestigt ist;
die Heizvorrichtung eine obere Abdeckplatte (21), eine obere Heizeinrichtung (22), eine Halterung (23), eine untere Heizeinrichtung (24), und eine Basis (25) aufweist; wobei die Halterung (23) zwischen der oberen Heizeinrichtung (22) und der unteren Heizeinrichtung (24) angeordnet ist; die obere Heizeinrichtung (22) unter einem mittigen Bereich der oberen Abdeckplatte (21) angeordnet ist; die untere Heizeinrichtung (24) über einem mittigen Bereich des Basis (25) angeordnet ist; die obere Heizeinrichtung (22) und die untere Heizeinrichtung (24) jeweils eine Vielzahl von unabhängigen kleinen Widerstandsdrähten (28) aufweisen, und eine zweite speicherprogrammierbare Steuerung (26, PLC) mit den kleinen Widerstandsdrähten (28) über einen Temperatursensor (27) verbunden ist, wobei die zweite speicherprogrammierbare Steuerung (26, PLC), der Temperatursensor und die kleinen Widerstandsdrähte die Heizvorrichtung steuern, um das PEEK-Material zu erhitzen, so dass dieses einen Erweichungspunkt von 260 +/- 10 °C erreicht.

## Revendications

1. Procédé de préparation d'os de crâne cérébral, le procédé comprenant les étapes pour :
a) sélectionner de la polyétheréthercétone (PEEK) comme matière première en fonction de données de tomodensitométrie (CT) d'un patient ;
b) chauffer la matière PEEK dans un dispositif chauffant pour atteindre un point de ramollissement de 260 ±10 °C ;
c) presser à chaud la matière PEEK chauffée obtenue à l'étape b) dans un moule de formage, refroidir et mettre en forme la matière PEEK pour donner une pièce brute ;
d) cuire la pièce brute, et placer la pièce brute dans un thermostat pour éliminer la contrainte interne et la résilience ;
e) éliminer les cristaux et impuretés superficiels de la pièce brute issue du pressage à chaud ;
f) traiter mécaniquement la pièce brute en fonction des données CT du patient pour donner un produit présentant une taille et une forme souhaitées, et
g) laver, désinfecter et emballer le produit,
le dispositif chauffant chauffe avec discrimination différentes zones de la matière PEEK pour satisfaire aux exigences de température et de force de compression pour la déformation de matière en fonction des données CT du patient ; les zones nécessitant une déformation relativement grande sont chauffées à une température relativement élevée, et les zones nécessitant une déformation relativement petite sont chauffées à une température relativement basse ;
le moule de formage comprend un moule supérieur comprenant un module d'aiguille supérieur et un moule inférieur comprenant un module d'aiguille inférieur ; le moule d'aiguille inférieur est commandé par un automate programmable (PLC) destiné à une régulation et une localisation d'espace en fonction des données CT ; le module d'aiguille supérieur est également régulé de manière correspondante, et la matière PEEK chauffée est placée sur le moule supérieur et traitée par les modules d'aiguille supérieur et inférieur pour donner la pièce brute.

2. Dispositif de préparation d'os de crâne cérébral, comprenant : un moule de formage et un dispositif de chauffage, **caractérisé en ce que** le moule de formage comprend une base de moule supérieur (11), un moule supérieur (12), un dispositif de localisation (13), un moule inférieur (14), une base de moule inférieur (15), un châssis de base (16) ; le moule supérieur (12) est fixé sur la base de moule supérieur (11) ; la base de moule inférieur (15) est fixée sur le châssis de base (16) ; le moule inférieur (14) est fixé sur la base de moule inférieur (15), et une extrémité inférieure du dispositif de localisation (13) est fixée sur le châssis de base (16) ; la base de moule supérieur (11) est fixée sur le dispositif de localisation (13), et une extrémité arrière de la base de moule supérieur (11) est située dans une rainure de coulissement du dispositif de localisation (13) ; le moule supérieur (12) et le moule inférieur (14) comprennent chacun une pluralité de modules d'aiguille (18) ; les modules d'aiguille (18) sont commandés par un premier PLC (19) par le biais d'un capteur de déplacement (20) ;
le dispositif de localisation (13) est perpendiculaire au châssis de base (16);
la base de moule inférieur (15) est fixée sur le châssis de base (16) par le biais d'un boulon verrou (17) ;
le dispositif chauffant comprend une plaque de recouvrement supérieure (21), un élément chauffant supérieur (22), un palier support (23), un élément chauffant inférieur (24), et une base (25) ; le palier support (23) est disposé entre l'élément chauffant supérieur (22) et l'élément chauffant inférieur (24) ; l'élément chauffant supérieur (22) est disposé sous une zone centrale de la plaque de recouvrement supérieure (21) ; l'élément chauffant inférieur (24) est disposé sur une zone centrale de la base (25) ; l'élément chauffant supérieur (22) et l'élément chauffant inférieur (24) comprennent chacun une pluralité de fils de faible résistance indépendants (28), et un second PLC (26) est connecté aux fils de faible résistance (28) par le biais d'un capteur de température (27), dans lequel le second PLC (26), le capteur de température et les fils de faible résistance commandent le dispositif chauffant pour chauffer la matière PEEK et atteindre un point de ramollissement de 260 ±10 °C.
